# EUROPÄISCHE PATENTANMELDUNG

(11) **EP 3 978 383 A1**
(43) Veröffentlichungstag der Anmeldung: **06.04.2022**
(21) Anmeldenummer: 20199808.5
(22) Anmeldetag: 02.10.2020
(51) Int. Cl.: B65D 30/00, B65D 33/00, B01L 3/00, A61L 2/26, A61L 2/07

(54) **VORRICHTUNG ZUM BEREITSTELLEN VON AUTOKLAVIERBAREN REAKTIONSGEFÄSSEN**

(71) Anmelder: Eppendorf AG, 22339 Hamburg (DE)
(72) Erfinder: Holl, Kai, 22359 Hamburg (DE); Hübler, Diana, 25337 Elmshorn (DE); Otto, Benjamin, 22175 Hamburg (DE); Grzeskowiak, Rafal, 22949 Hamburg (DE)
(74) Vertreter: Hauck Patentanwaltspartnerschaft mbB

(57) **Zusammenfassung**

Vorrichtung zum Bereitstellen von autoklavierbaren Reaktionsgefäßen für Arbeiten im Labor umfassend eine zum Autoklavieren zumindest teilweise wasserdampfdurchlässige Verpackung aus einem keimundurchlässigen, autoklavierbaren Material und eine Vielzahl in der Verpackung angeordnete Reaktionsgefäße.

## Beschreibung

Die Erfindung betrifft ein System zum Bereitstellen von autoklavierten Reaktionsgefäßen für Arbeiten im Labor.

Reaktionsgefäße aus Kunststoff für einmaligen Gebrauch (Disposables) werden insbesondere in wissenschaftlichen und industriellen Labors mit medizinischen, molekularbiologischen und pharmazeutischen Anwendungsgebieten zum Behandeln von Proben verwendet. Reaktionsgefäße (Mikroreaktionsgefäße) werden in verschiedenen Größen angeboten, vorzugsweise im Bereich von 0,1 ml bis 5,0 ml. Sie sind meist mit einem aufklemmbaren, aufschnappbaren bzw. aufschraubbaren Deckel versehen und werden auch als "Deckelgefäße" bezeichnet.

In den meisten Laboren werden Reaktionsgefäße standardmäßig vor der Verwendung autoklaviert. Dazu muss das Reaktionsgefäß aus einer Primärverpackung in ein Becherglas oder einen anderen Autoklavierbehälter überführt werden. Dieses Überführen wird von den meisten Anwendern als lästig und ineffizient wahrgenommen. Das Becherglas wird anschließend mit Alufolie verschlossen und autoklaviert. Die Verwendung von Alufolie als Verschluss ist zwar gängige Praxis, bietet jedoch keinen ausreichenden Sterilitätsschutz. Zudem wird die Alufolie bei mehrfachem Öffnen und Schließen des Becherglases oft beschädigt. Auch wird die Verwendung von Alufolie aus umwelttechnischen Gründen kritisch gesehen. Zudem ist nachteilig, dass die Autoklavierbehälter nach dem Autoklavieren aufgrund der oft hohen Restfeuchte lange getrocknet werden müssen. Außerdem ist die sterile Entnahme der Reaktionsgefäße aus dem Becherglas schwierig. Bei der Lagerung nehmen Bechergläser große Teile des wertvollen Laborarbeitsplatzes weg, da sie nicht stapelbar sind. Zudem können Gefäße beim "Umschütten" leicht kontaminieren und sind zu verwerfen.

Davon ausgehend liegt der Erfindung die Aufgabe zugrunde, Reaktionsgefäße in einer Weise bereitzustellen, die es ermöglicht, diese einfacher und schneller zu autoklavieren und besser gegen Kontamination zu schützen.

Die Aufgabe wird durch eine Vorrichtung zum Bereitstellen von autoklavierbaren Reaktionsgefäßen gemäß Anspruch 1 gelöst. Vorteilhafte Ausführungsarten der Erfindung sind in den Unteransprüchen und in der nachfolgenden Beschreibung angegeben.

Die erfindungsgemäße Vorrichtung zum Bereitstellen von autoklavierbaren Reaktionsgefäßen für Arbeiten im Labor umfasst eine zum Autoklavieren zumindest teilweise wasserdampfdurchlässige Verpackung aus einem keimundurchlässigen, autoklavierbaren Material, und eine Vielzahl in der Verpackung angeordnete Reaktionsgefäße.

Bei der erfindungsgemäßen Vorrichtung werden dem Anwender Reaktionsgefäße in einer zumindest teilweise wasserdampfdurchlässigen Verpackung aus einem keimundurchlässigen, autoklavierbaren Material zur Verfügung gestellt. Bei dem autoklavierbaren Material handelt es sich um ein Material, dass einer Dampfsterilisation mittels Wasserdampf in einem Autoklaven Stand hält. Die Dampfsterilisation von Reaktionsgefäßen erfolgt vorzugsweise bei einer Temperatur von 121°C über eine Sterilisierzeit von 15 Minuten bei einem absoluten Druck von 2,1 bar. Gemäß einer bevorzugten Ausführungsart der Erfindung besteht die Verpackung aus einem Material, dass den vorgenannten Bedingungen standhält. Zur Erhöhung der Sicherheit werden die Sterilisierzeiten meistens verlängert, und zwar auf 20 Minuten bei 121°C und vorzugsweise hält das Material der Verpackung auch den verlängerten Sterilisationszeiten stand. Aufgrund der Wasserdampfdurchlässigkeit der Verpackung kann der Anwender die Reaktionsgefäße direkt in der Verpackung autoklavieren, sodass das lästige Überführen der Reaktionsgefäße vor dem Autoklavieren in ein Autoklavierbehältnis entfällt. Aufgrund der Keimundurchlässigkeit des Materials der Verpackung kann der Anwender die Reaktionsgefäße nach dem Autoklavieren in der Verpackung steril lagern. Unter Keimundurchlässigkeit wird verstanden, dass das Material der Verpackung eine Barriere für Keime bildet, welche den Durchgang von Keimen verhindert oder auf ein vernachlässigbares Ausmaß begrenzt. Als keimundurchlässig werden insbesondere homogene Folien aus Kunststoff oder Aluminium mit geschlossener Oberfläche ohne Poren oder andere Öffnungen und poröse Folien, Vliesstoffe (Nonwoven) oder Papiere mit einem Porendurchmesser von maximal 1 µm, vorzugsweise von maximal 0,6 µm, besonders bevorzugt von maximal 0,2 µm, bezeichnet. Die homogenen Folien mit geschlossener Oberfläche lassen Keime nicht und die porösen Materialien allenfalls in einem vernachlässigbaren Ausmaß durch, da Keime im Allgemeinen einen Durchmesser von mindestens etwa 1 µm aufweisen. Für die Entnahme von Reaktionsgefäßen aus der Verpackung muss diese geöffnet werden. Die Reaktionsgefäße werden nach dem Öffnen der Verpackung im Allgemeinen in kurzer Zeit verbraucht. Vielfach werden die Reaktionsgefäße in der Sicherheitswerkbank (Bench) oder einer anderen sterilen oder keimarmen Umgebung aufbewahrt. Deshalb kann es ausreichend sein, die Reaktionsgefäße bis zum Verbrauch in der geöffneten Verpackung zu verwahren, ohne dass eine unzulässige Verkeimung eintritt. Vorzugsweise wird die Verpackung bis zum Verbrauch der Reaktionsgefäße in einem schließbaren Behälter aufbewahrt, um eine Verkeimung in der geöffneten Verpackung zu verhindern. Dies wird weiter unten noch näher erläutert.

Gemäß einer weiteren Ausführungsart sind die Reaktionsgefäße Deckelgefäße und mit geöffnetem Deckel in der Verpackung angeordnet. Gemäß einer weiteren Ausführungsart haben die Reaktionsgefäße eine Größe im Bereich von 0,1 ml bis 5,0 ml. Gemäß einer weiteren Ausführungsart haben sämtliche Reaktionsgefäße in der Verpackung dieselbe Größe.

Gemäß einer bevorzugten Ausführungsart werden dem Anwender die Reaktionsgefäße in nicht sterilisiertem Zustand zur Verfügung gestellt und die Reaktionsgefäße werden erst vom Anwender sterilisiert. Alternativ wird die Verpackung dem Anwender mit bereits sterilisierten Reaktionsgefäßen zur Verfügung gestellt und die Reaktionsgefäße werden von dem Anwender nochmals sterilisiert, beispielsweise wenn aufgrund längerer Lagerzeiten hinreichende Keimfreiheit der Reaktionsgefäße nicht mehr sichergestellt ist.

Gemäß einer weiteren Ausführungsart der Erfindung umfasst die Vorrichtung einen zum Aufnehmen der Verpackung ausgebildeten Behälter, sodass die Verpackung darin eingesetzt werden kann, um zu verhindern, dass Keime in die geöffnete Verpackung eindringen. Der Behälter hat einen öffenbaren und wiederverschließbaren Deckel zum Schutz der Reaktionsgefäße nach dem Öffnen der Verpackung. Die Verpackung wird vorzugsweise nach dem Einsetzen in den Behälter geöffnet. Grundsätzlich kann sie aber auch vorher geöffnet werden. Nach dem Einsetzen der Verpackung kann der Deckel des Behälters geschlossen werden. Dies verhindert, dass die Reaktionsgefäße in der geöffneten Verpackung verkeimen. Für die Entnahme von Reaktionsgefäßen muss der Deckel des Behälters geöffnet werden. Da die Reaktionsgefäße nach dem Öffnen der Verpackung im allgemeinen in kurzer Zeit verbraucht und vielfach in der Sicherheitswerkbank (Bench) oder einer anderen sterilen oder keimarmen Umgebung aufbewahrt werden, ist es nicht notwendig, dass der geschlossene Behälter keimundurchlässig ist. Die Erfindung umfasst aber auch Ausführungsarten mit einem im geschlossenen Zustand keimundurchlässigen Behälter. Die Verpackung mit den darin enthaltenen Reaktionsgefäßen und der zum Aufnehmen der Verpackung ausgebildete Behälter bilden gemeinsam ein System zum Bereitstellen von autoklavierbaren Reaktionsgefäßen.

Gemäß einer weiteren Ausführungsart weist die Verpackung eine öffenbare Abdeckung auf. Die Abdeckung kann aus demselben Material wie die übrigen Teile der Verpackung bestehen oder aus einem anderen Material. Die Abdeckung und die übrigen Teile der Verpackung oder nur die Abdeckung oder nur die übrigen Teile der Verpackung sind aus einem wasserdampfdurchlässigen Material gebildet.

Gemäß einer weiteren Ausführungsart ist die Verpackung ein Beutel aus einem Vliesstoff aus Kunststofffasern oder aus einem Papier. Für den Vliesstoff oder das Papier werden wasserdampfdurchlässige, keimundurchlässige und autoklavierbare Materialien verwendet. Gemäß einer weiteren Ausführungsart hat der Vliesstoff oder das Papier eine Porengröße von maximal 1 µm, vorzugsweise von maximal 0,6 µm, besonders bevorzugt von maximal 0,2 µm. Zum Bilden des Beutels geeignete Papiere werden beispielsweise als Sterilisationspapier für das Einschlagen von Instrumenten im medizinischen Bereich oder als Siegelpapier zum Verpacken von Operationsbesteck oder Spritzen in Tiefziehfolien verwendet (medizinisches Papier). Gemäß einer bevorzugten Ausführungsart ist das flexible Flachmaterial ein Polyethylenvlies. Gemäß einer weiteren Ausführungsart ist das flexible Flachmaterial ein hochdichtes Polyethylenvlies. Gemäß einer weiteren Ausführungsart ist das flexible Flachmaterial ein Polyethylen-Spinnvlies. Gemäß einer weiteren Ausführungsart ist das flexible Flachmaterial ein hochdichtes Polyethylenvlies, das von der Firma Dupont unter der Markenbezeichnung Tyvek^{®} vermarktet wird. Die Reaktionsgefäße sind in dem Beutel in loser Schüttung verpackt. Nach dem Autoklavieren und Trocknen kann der Beutel in einen dazu passenden Behälter eingesetzt werden. Zum Entnehmen von Reaktionsgefäßen kann der Beutel beispielsweise mittels einer Schere aufgeschnitten oder entlang einer Siegelnaht aufgerissen werden, die hierfür als Schwächungslinie ausgebildet sein kann.

Der Beutel ist beispielsweise als Schlauchbeutel oder Kreuzbodenbeutel ausgebildet und am Rand jeder Öffnung durch eine Siegelnaht geschlossen. Vorzugsweise ist die Siegelnaht keimundurchlässig ausgebildet. Für die Keimundurchlässigkeit der Siegelnaht gelten die oben beschriebenen Anforderungen für die Keimundurchlässigkeit des Materials der Verpackung entsprechend. Dies gilt auch für weitere Ausführungsarten der Erfindung, die eine keimundurchlässige Siegelnaht aufweisen.

Gemäß einer weiteren Ausführungsart weist die Verpackung einen Beutel aus Vliesstoff, Papier, Kunststofffolie oder Aluminiumfolie und eine Abdeckung in Form eines auf einen Rand einer Öffnung des Beutels vollständig umlaufend aufgesiegelten, flexiblen Flachmaterials auf. Hierbei kann der Beutel und die Abdeckung oder nur der Beutel oder nur die Abdeckung wasserdampfdurchlässig ausgebildet sein. Die Reaktionsgefäße sind in der Verpackung in loser Schüttung verpackt. Nach dem Autoklavieren und Trocknen kann die Verpackung in einen dazu passenden Behälter eingesetzt werden. Durch Ablösen der aufgesiegelten Abdeckung kann der Beutel geöffnet werden. Nach Entnahme von Reaktionsgefäßen wird der Deckel des Behälters geschlossen.

Gemäß einer weiteren Ausführungsart ist das flexible Flachmaterial der Abdeckung ein wasserdampfdurchlässiger, keimundurchlässiger und autoklavierbarer Vliesstoff aus Kunststofffasern oder ein Papier. Das Papier ist beispielsweise ein Sterilisationspapier oder ein medizinisches Papier. Gemäß einer weiteren Ausführungsart hat der Vliesstoff oder das Papier Poren mit einer Porengröße von maximal 1 µm, vorzugsweise von maximal 0,6 µm, besonders bevorzugt von maximal 0,2 µm. Gemäß einer bevorzugten Ausführungsart ist das flexible Flachmaterial ein Polyethylenvlies. Gemäß einer weiteren Ausführungsart ist das flexible Flachmaterial ein hochdichtes Polyethylenvlies. Gemäß einer weiteren Ausführungsart ist das flexible Flachmaterial ein Polyethylen-Spinnvlies. Gemäß einer weiteren Ausführungsart ist das flexible Flachmaterial ein hochdichtes Polyethylenvlies, das von der Firma Dupont unter der Markenbezeichnung Tyvek^{®} vermarktet wird. Gemäß einer weiteren Ausführungsart ist die Abdeckung mittels einer keimundurchlässigen Siegelnaht auf dem Rand der Öffnung des Beutels vollständig umlaufend aufgesiegelt.

Gemäß einer weiteren Ausführungsart ist der Beutel ein Blister mit einem vollständig umlaufenden Randflansch an einer Öffnung, auf den die Abdeckung aufgesiegelt ist. Der Blister (Sichtverpackung) ist ein Formteil aus einer durchsichtigen Kunststofffolie, durch die hindurch die Reaktionsgefäße von außen sichtbar sind. Der Blister kann durch Umformung thermoplastischer Kunststoffe unter Wärmeeinwirkung sowie mithilfe von Druckluft oder Vakuum (auch "Thermoformen", "Warmformen" oder "Tiefziehen" genannt) hergestellt werden. Gemäß einer weiteren Ausführungsart besteht der Blister aus einer homogenen Kunststofffolie mit geschlossener Oberfläche ohne Poren oder sonstige Öffnungen. Hierdurch ist der Blister keimundurchlässig ausgebildet.

Gemäß einer weiteren Ausführungsart weist die Verpackung eine steife Schale aus Kunststoff und einen steifen Deckel aus Kunststoff auf und ist bei geschlossener Verpackung ein um die Schale umlaufender Anlagebereich zwischen Deckel und Schale wasserdampfdurchlässig. Gemäß einer weiteren Ausführungsart hat die Schale eine geschlossene Oberfläche ohne Poren oder sonstige Öffnungen. Hierdurch ist die Schale keimundurchlässig. Gemäß einer weiteren Ausführungsart weist der Deckel eine geschlossene Oberfläche ohne Poren oder sonstige Öffnungen auf. Hierdurch ist der Deckel keimundurchlässig. Gemäß einer weiteren Ausführungsart ist auch der Deckel eine Schale. Die Ausführungsarten mit steifer Schale und steifem Deckel werden gemäß einer weiteren Ausführungsart durch Thermoformen hergestellt. Die Reaktionsgefäße werden in loser Schüttung in der Verpackung verpackt. Beim Autoklavieren kann der Wasserdampf durch den wasserdampfdurchlässigen Anlagebereich zwischen Deckel und Schale hindurchtreten. Die Erfindung umfasst Ausführungsarten, bei denen der Anlagebereich zwischen Deckel und Schale nicht keimundurchlässig, wie Deckel und Schale, ausgebildet ist. Die Sterilität der autoklavierten Reaktionsgefäße wird durch eine geringe Keimdurchlässigkeit des Anlagebereichs nicht beeinträchtigt, insbesondere wenn die Lagerzeiten kurz sind und die Lagerung in keimarmer Umgebung erfolgt.

Gemäß einer weiteren Ausführungsart ist der Anlagebereich keimundurchlässig ausgebildet.

Gemäß einer weiteren Ausführungsart umfasst der Anlagebereich flächig aneinander anliegende, umlaufende Randflansche von Schale und Deckel. Hierdurch kann eine verbesserte Abdichtung im Anlagebereich bis hin zur Keimundurchlässigkeit erreicht werden. Ferner kann der Anlagebereich beim Autoklavieren geringfügig geöffnet werden, um die Wasserdampfdurchlässigkeit zu gewährleisten, und kann der Anlagebereich nach dem Autoklavieren geschlossen werden.

Gemäß einer weiteren Ausführungsart umfasst die Verpackung eine Einrichtung zum Verriegeln von Schale und Deckel bei geschlossener Verpackung. Gemäß einer weiteren Ausführungsart ist die Einrichtung zum Verriegeln eine Schnappverbindung oder eine Klemmverbindung, die bei geschlossener Verpackung den Deckel an der Schale festhält.

Gemäß einer weiteren Ausführungsart ist die Schale über ein Filmscharnier mit dem Deckel verbunden. Gemäß einer weiteren Ausführungsart verbindet das Filmscharnier zwei benachbarte seitliche Ränder der umlaufenden Randflansche von Schale und Deckel miteinander.

Gemäß einer weiteren Ausführungsart ist die Schale und/oder der Deckel ein Tiefziehteil aus einer Kunststoffplatte oder einer Kunststoffbahn oder ein Spritzgussteil. Ein Tiefziehteil ist ein durch Thermoformen hergestelltes Bauteil und ein Spritzgussteil ist ein durch Spritzgießen hergestelltes Bauteil.

Gemäß einer weiteren Ausführungsart weist die Verpackung eine steife Schale aus Kunststoff mit einem vollständig umlaufenden Randflansch an einer Öffnung und eine Abdeckung in Form eines vollständig umlaufenden auf den Randflansch aufgesiegelten wasserdampfdurchlässigen, flexiblen Flachmaterials auf. Gemäß einer weiteren Ausführungsart hat die Schale eine geschlossene Oberfläche ohne Poren oder sonstige Öffnungen. Damit ist die Schale keimundurchlässig ausgebildet. Gemäß einer weiteren Ausführungsart besteht die Abdeckung aus einem wasserdampfdurchlässigen, keimundurchlässigen und autoklavierbaren Vliesstoff aus Kunststofffasern oder aus einem Papier. Gemäß einer weiteren Ausführungsart ist das Papier ein Sterilisationspapier oder ein medizinisches Papier. Gemäß einer weiteren Ausführungsart weist der Vliesstoff oder das Papier Poren mit einer Porengröße von maximal 1 µm, vorzugsweise von maximal 0,6 µm, besonders bevorzugt von maximal 0,2 µm auf. Gemäß einer bevorzugten Ausführungsart ist das flexible Flachmaterial ein Polyethylenvlies. Gemäß einer weiteren Ausführungsart ist das flexible Flachmaterial ein hochdichtes Polyethylenvlies. Gemäß einer weiteren Ausführungsart ist das flexible Flachmaterial ein Polyethylen-Spinnvlies. Gemäß einer weiteren Ausführungsart ist das flexible Flachmaterial ein hochdichtes Polyethylenvlies, das von der Firma Dupont unter der Markenbezeichnung Tyvek^{®} vermarktet wird. Gemäß einer weiteren Ausführungsart ist das Flachmaterial mittels einer keimundurchlässigen Siegelnaht auf den Randflansch aufgesiegelt. Die Reaktionsgefäße sind in der steifen Schale in loser Schüttung verpackt. Nach dem Autoklavieren kann die Verpackung direkt in den Behälter überführt werden. In dem Behälter wird die Abdeckung vom Anwender entfernt. Nach der Entnahme von Reaktionsgefäßen können restliche Reaktionsgefäße in der Schale durch Schließen des Deckels geschützt werden. Nach Entnahme sämtlicher Reaktionsgefäße kann die Schale aus dem Behälter entnommen und durch eine weitere autoklavierte Verpackung ersetzt werden.

Gemäß einer weiteren Ausführungsart weist die Verpackung eine steife Schale aus einem Kunststoff mit einer daran angelenkten, steifen Haube aus Kunststoff auf, ist der Deckel des Behälters ein Klappdeckel und sind die Haube und der Deckel ausgebildet, bei in dem Behälter eingesetzter Verpackung Haube und Klappdeckel miteinander zu verriegeln, sodass durch Öffnen des Klappdeckels zugleich die Haube der Verpackung öffenbar ist. Gemäß einer weiteren Ausführungsart weist die Verpackung eine geschlossene Oberfläche ohne Poren oder sonstige Öffnungen auf. Gemäß einer weiteren Ausführungsart liegen bei geschlossener Verpackung Deckel und Schale in einem um die Schale umlaufenden, wasserdampfdurchlässigen Anlagebereich aneinander an. Hierdurch wird ein Autoklavieren der Reaktionsgefäße in der Verpackung bei geschlossenem Deckel ermöglicht. Ferner kann der Deckel zur Gewährleistung einer hinreichenden Wasserdampfdurchlässigkeit beim Autoklavieren geöffnet werden. Gemäß einer weiteren Ausführungsart ist der Anlagebereich keimundurchlässig oder in einem geringfügigen Ausmaß keimdurchlässig.

Gemäß einer weiteren Ausführungsart ist die Schale und/oder die Haube ein Spritzgussteil.

Gemäß einer weiteren Ausführungsart trägt der Deckel eine Beschriftung und/ oder die Schale einen Farbcode, wobei die Beschriftung die Größe der Reaktionsgefäße in der Verpackung angibt und/oder der Farbcode die Größe der Reaktionsgefäße in der Verpackung repräsentiert.

Gemäß einer weiteren Ausführungsart ist die Verbindung zwischen Haube und Klappdeckel so ausgebildet, wie in der EP 2 533 109 B1 für die Verbindung zwischen einem Klappdeckel eines Behälters und einer Abdeckung einer Nachfüllpackung für Pipettenspitzen beschrieben.

Die Reaktionsgefäße sind in loser Schüttung in der Verpackung angeordnet. Das Autoklavieren kann bei leicht geöffnetem Deckel oder bei geschlossenem Deckel ausgeführt werden, wenn der Anlagebereich wasserdampfdurchlässig ist. Nach dem Autoklavieren wird die Verpackung bei geschlossenem Deckel in den Behälter überführt und aufgrund der Verriegelung von Haube und Deckel öffnet die Verpackung automatisch beim Öffnen des Behälters. Auch kann die Verpackung durch Schließen des Deckels geschlossen werden. Aufgrund ihrer aufwändigeren Konstruktion wird die Verpackung bevorzugt mehrfach zum Beladen des Behälters mit Reaktionsgefäßen verwendet.

Gemäß einer weiteren Ausführungsart besteht die Schale der Verpackung aus PE, PP oder COC und/oder besteht der Deckel der Verpackung aus PE, PP oder COC.

Gemäß einer weiteren Ausführungsart bestehen die Kunststofffasern des Vliesstoffes aus PE, PP oder aus PE und PP (z. B. Bikomponentenfasern).

Gemäß einer weiteren Ausführungsart weist die Verpackung einen Feuchtigkeitsindikator zur Detektion der Restfeuchte auf und/oder weist die Verpackung einen Autoklavierindikator zur Detektion eines Autoklavierens unter vorgegebenen Bedingungen auf. Der Autoklavierindikator zeigt an, ob die Verpackung schon Autoklaviert wurde und der Feuchtigkeitsindikator, ob die Produkte erfolgreich getrocknet wurden bzw. noch trocken sind.

Gemäß einer weiteren Ausführungsart ist der Behälter steif und ist der Deckel des Behälters steif ausgebildet.

Gemäß einer weiteren Ausführungsart besteht der Behälter aus einem autoklavierbaren Material. Dies ermöglicht ein Autoklavieren von Behälter und Deckel vor Aufnahme der Benutzung.

Gemäß einer weiteren Ausführungsart ist der Behälter keimundurchlässig, sodass bei geschlossenem Behälter keine Keime ins Innere des Behälters eindringen können.

Ferner betrifft die Erfindung die Verwendung der Vorrichtung nach einem der Ansprüche 1 bis 17 oder einer der zuvor beschriebenen weiteren Ausführungsarten, bei der die Verpackung und die darin enthaltenen Reaktionsgefäße autoklaviert werden. Gemäß einer Ausführungsart wird die Verpackung mit den autoklavierten Reaktionsgefäßen in den Behälter eingesetzt, die Verpackung geöffnet und nach Entnahme von Reaktionsgefäßen aus der Verpackung der Deckel des Behälters geschlossen.

Gemäß einer weiteren Ausführungsart wird die Verpackung gemäß einem der Ansprüche 1 bis 12 nach Entnahme sämtlicher Reaktionsgefäße nicht wiederverwendet.

Gemäß einer weiteren Ausführungsart wird die Verpackung nach einem der Ansprüche 13 bis 15 nach Entnahme sämtlicher Reaktionsgefäße erneut mit Reaktionsgefäße befüllt und wiederverwendet.

In den Figuren sind Ausführungsbeispiele der Erfindung dargestellt. In den Figuren zeigen:
- FIG. 1.1, 1.2: eine Reaktionsgefäße enthaltende Verpackung mit einem Beutel und einem aufgesiegelten Papier in einer Perspektivansicht schräg von oben und von der Seite (FIG. 1.1) und in einem Vertikalschnitt (FIG. 1.2);
- FIG. 2.1, 2.2: eine Reaktionsgefäße enthaltende Verpackung mit einer steifen Schale und einem steifen Deckel in einer Perspektivansicht schräg von oben und von der Seite (FIG. 2.1) und in einem Vertikalschnitt (FIG. 2.2);
- FIG. 3.1, 3.2: eine Reaktionsgefäße enthaltende Verpackung mit einer steifen Schale und einem aufgesiegelten Papier in einer Perspektivansicht schräg von oben und von der Seite (FIG. 3.1) und in einem Vertikalschnitt (FIG. 3.2);
- FIG. 4.1, 4.2: eine Reaktionsgefäße enthaltende Verpackung mit einer steifen Schale und einer steifen Haube in einer Perspektivansicht schräg von oben und von der Seite (FIG. 4.1) und in einem Vertikalschnitt (FIG. 4.2);
- FIG. 5: ein Behälter zum Aufnehmen einer Verpackung im geöffneten Zustand in einer Perspektivansicht schräg von vorn und von der Seite.

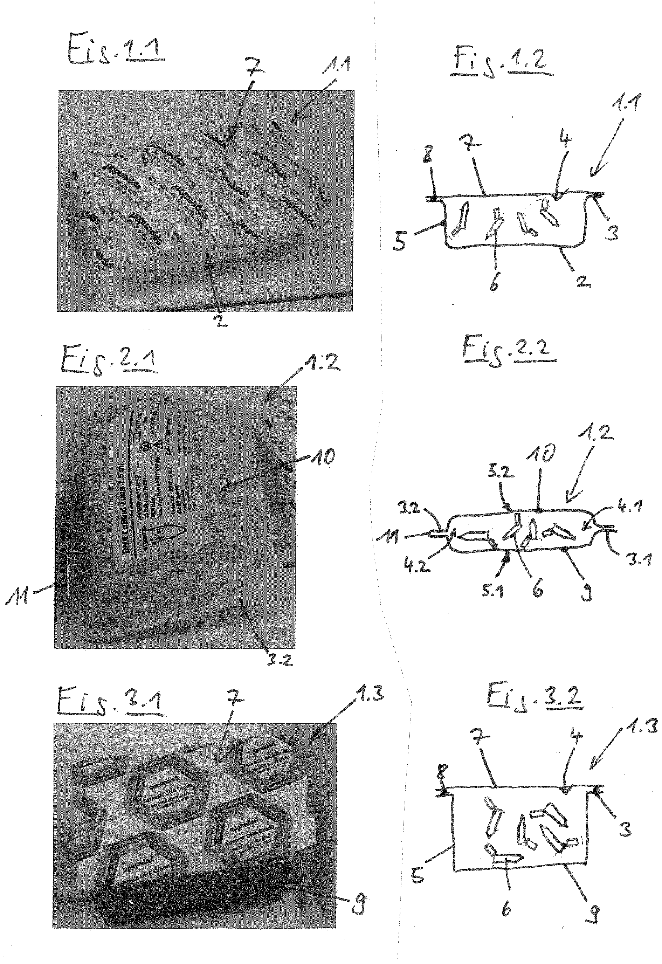

Gemäß FIG. 1 umfasst eine Verpackung 1.1 einen Beutel 2 in Form eines Blisters aus einem Kunststoff mit einem vollständig umlaufenden Randflansch 3 an einer Öffnung 4. Der Beutel 2 hat eine geschlossene Oberfläche 5. Der Beutel ist mit Reaktionsgefäßen 6 befüllt. Auf den Randflansch 3 ist um die Öffnung 4 umlaufend ein wasserdampfdurchlässiges und keimundurchlässiges Papier 7 aufgesiegelt. Die Siegelnaht 8 ist ebenfalls keimundurchlässig ausgebildet. Für die Verpackung 1.1 werden ausschließlich Materialien verwendet, die mittels Wasserdampf autoklavierbar sind, sodass sie den dabei auftretenden Temperaturen und Drucken während der für die Sterilisierung der enthaltenen Reaktionsgefäße 6 erforderlichen Sterilisationszeit standhalten. Die Siegelnaht 8 ist so ausgebildet, dass das Papier 7 von Hand ohne große Anstrengung vom Beutel 2 abgezogen werden kann. Die Verpackung 1.1 ist besonders kostengünstig herstellbar und für einmaligen Gebrauch bestimmt.

FIG. 2 zeigt eine Verpackung 1.2, die eine steife Schale 9 aus einem Kunststoff mit einem um die Öffnung 4.1 umlaufenden Randflansch 3.1 und eine geschlossene Oberfläche 5.1 aufweist. Ferner weist die Verpackung 1.2 einen steifen, haubenartigen Deckel 10 aus Kunststoff mit einem um eine Öffnung 4.2 umlaufenden Randflansch 3.2 und einer geschlossenen Oberfläche 5.2 auf. Die Schale 9 und der Deckel 10 sind an geradlinigen Randabschnitten der Randflansche 3.1, 3.2 durch ein Filmscharnier 11 miteinander verbunden. Die Schale 9 und der Deckel 10 sind durch Thermoformen oder Spritzgießen hergestellt. In der Verpackung 1.2sind eine Vielzahl Reaktionsgefäße 6 angeordnet. Die Verpackung 1.2 ist durch Verschweißen oder Versiegeln der Randflansche 3.1, 3.2 geschlossen. Die Randflansche 3.1, 3.2 liegen so aneinander an, dass im Anlagebereich Wasserdampf hindurchtreten kann. Diese Ausführungsart ist ebenfalls kostengünstig herstellbar und für einmaligen Gebrauch bestimmt. Infolgedessen kann die Verpackung 1.2 nach Entnahme sämtlicher Reaktionsgefäße 6 entsorgt werden.

In FIG. 3 ist eine Verpackung 1.3 mit einer steifen Schale 9 aus einem Kunststoff mit einem vollständig umlaufenden Randflansch 3 an einer Öffnung 4 und einer geschlossenen Oberfläche 5 gezeigt. Auf den Randflansch 3 ist ein keimundurchlässiges und wasserdampfdurchlässiges Papier 7 umlaufend aufgesiegelt. Die Wasserdampfdurchlässigkeit ermöglicht eine Wasserdampfsterilisierung im Autoklaven. Mehrere Verpackungen 1.3 sind aufeinander stapelbar. Die Verpackung 1.3 kann in verschiedenen Reinheitsgraden bereitgestellt werden. Die Verpackung 1.3 ist für Einmalbenutzung konzipiert, sodass sie nach Entnahme sämtlicher Reaktionsgefäße 6 entsorgt werden kann.

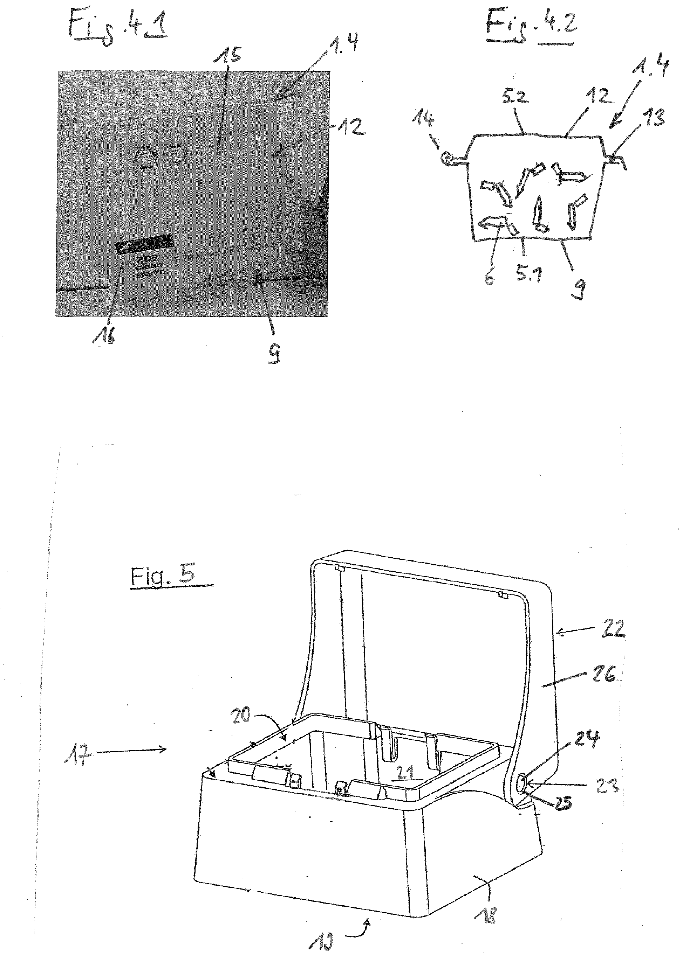

In FIG. 4 ist eine Verpackung 1.4 gezeigt, die eine steife Schale 9 aus Kunststoff und eine daran angelenkte, steife Haube 12 aus Kunststoff aufweist. Der Anlagebereich 13 von Schale und Haube 12 ist wasserdampfdurchlässig. Schale 9 und Haube 12 sind Spritzgussteile. Sie sind durch ein Gelenk 14 miteinander verbunden. Schale 9 und Haube 12 haben jeweils eine geschlossene Oberfläche 5.1, 5.2.

Die Verpackung 1.4 ist mit Reaktionsgefäßen 6 befüllt. Die Haube 12 trägt eine Beschriftung 15 und die Schale 9 einen Farbcode 16, welche die Größe der Reaktionsgefäße 6 in der Verpackung 1.4 angegeben.

Aufgrund der Wasserdampfdurchlässigkeit des Anlagebereiches 13 ermöglicht die Verpackung 1.4 ebenfalls ein Dampfsterilisieren der enthaltenen Reaktionsgefäße 6 und der Verpackung 1.4 selber. Aufgrund der steifen Ausbildung ist die Verpackung 1.4 für Mehrfachbenutzung geeignet, sodass nach Entnahme der Reaktionsgefäße 6 diese erneut mit Reaktionsgefäßen 6 gefüllt und wiederverwendet werden kann.

FIG. 5 zeigt einen Behälter 17, der zur Aufnahme der zuvor beschriebenen Verpackungen 1.1 bis 1.4 bestimmt ist. Der Behälter 17 ist kastenförmig mit Seitenwänden 18 und einer Bodenwand 19 sowie einer Öffnung 20 an der Oberseite zu dem von den Seitenwänden 18 und der Bodenwand 19 begrenzten Innenraum 21 ausgebildet. Ein Deckel 22 ist über ein Schwenkgelenk 23 mit dem Behälter 17verbunden. Das Schwenkgelenk 23 ist durch nach außen von zwei Seitenwänden 18 des Behälters 17 vorstehende Lagerzapfen 24 und zwei Lagerbohrungen 25 in zwei gegenüberliegenden Seitenwänden 26 des haubenartigen Deckels 22 gebildet. Vorzugsweise ist der Behälter 17 ebenfalls aus einem autoklavierbaren Kunststoff hergestellt.

Weitere Einzelheiten des Behälters 17 sind in der Beschreibung des Ausführungsbeispiels von FIG. 3.1 der EP 2 535 109 B1 beschrieben. In der Patentschrift wird der Behälter für einen anderen Einsatzzweck verwendet und kann im Rahmen der Erfindung einer weiteren Verwendung zugeführt werden.

Jede der Verpackungen 1.1 bis 1.4 von FIG. 1 bis 4 sind in den Behälter 17 einsetzbar. In dem Behälter 17 können die Verpackungen 1.1 bis 1.4 an einem Arbeitsplatz für die Entnahme von Reaktionsgefäßen 6 bereitgehalten werden. Die Reaktionsgefäße 6 in der geöffneten Verpackung 1.1 bis 1.4 können durch Zuklappen des Deckels 22 geschützt werden.

## Patentansprüche

1. Vorrichtung zum Bereitstellen von autoklavierbaren Reaktionsgefäßen für Arbeiten im Labor umfassend eine zum Autoklavieren zumindest teilweise wasserdampfdurchlässige Verpackung aus einem keimundurchlässigen, autoklavierbaren Material und eine Vielzahl in der Verpackung angeordnete Reaktionsgefäße.

2. Vorrichtung nach Anspruch 1 umfassend einen zum Aufnehmen der Verpackung ausgebildeten Behälter mit einem öffenbaren und wiederverschließbaren Deckel zum Schutz der Reaktionsgefäße nach dem Öffnen der Verpackung.

3. Vorrichtung nach Anspruch 1 oder 2, bei dem die Verpackung eine öffenbare Abdeckung aufweist.

4. Vorrichtung nach Anspruch 1 oder 2, bei dem die Verpackung ein Beutel aus einem wasserdampfdurchlässigen Vliesstoff aus Kunststofffasern oder einem Papier ist.

5. Vorrichtung nach einem der Ansprüche 1 bis 3, bei dem die Verpackung einen Beutel aus Vliesstoff, Papier, Kunststofffolie oder Aluminiumfolie und eine Abdeckung in Form eines auf einen Rand einer Öffnung des Beutels vollständig umlaufend aufgesiegelten, flexiblen Flachmaterials aufweist.

6. Vorrichtung nach Anspruch 5, bei dem die Abdeckung wasserdampfdurchlässig ist.

7. Vorrichtung nach Anspruch 5 oder 6, bei dem der Beutel ein Blister mit einem vollständig umlaufenden Randflansch an einer Öffnung ist, auf den das flexible Flachmaterial aufgesiegelt ist und/oderbei dem das flexible Flachmaterial ein Vliesstoff aus Kunststofffasern oder ein Papier ist.

8. Vorrichtung nach einem der Ansprüche 1 bis 3, bei dem die Verpackung eine steife Schale aus Kunststoff und eine Abdeckung in Form eines steifen Deckels aus Kunststoff aufweist und bei der geschlossenen Verpackung ein um die Schale umlaufender Anlagebereich zwischen Deckel und Schale wasserdampfdurchlässig ist.

9. Vorrichtung nach Anspruch 8, bei dem der Anlagebereich aneinander anliegende Randflansch von Schale und Deckel umfasst und/oderbei dem die Schale über ein Filmscharnier mit dem Deckel verbunden ist.

10. Vorrichtung nach Anspruch 8 oder 9, bei dem die Schale und/oder der Deckel ein Tiefziehteil aus einer Kunststoffplatte oder einer Kunststoffbahn oder ein Spritzgussteil ist.

11. Vorrichtung nach einem der Ansprüche 1 bis 3, bei dem die Verpackung eine steife Schale aus einem Kunststoff mit einem vollständig umlaufenden Randflansch an einer Öffnung und eine Abdeckung in Form eines vollständig umlaufend auf den Randflansch aufgesiegelten wasserdampfdurchlässigen, flexiblen Flachmaterials aufweist.

12. Vorrichtung nach Anspruch 11, bei dem die Schale ein Tiefziehteil aus einer Kunststoffplatte oder einer Kunststoffbahn oder ein Spritzgussteil ist und/oder bei dem das flexible Flachmaterial ein Vliesstoff aus Kunststofffasern oder ein Papier ist.

13. Vorrichtung nach einem der Ansprüche 1 bis 3, bei dem die Verpackung eine steife Schale aus Kunststoff mit einer daran angelenkten, steifen Haube aus Kunststoff aufweist, der Deckel des Behälters ein Klappdeckel ist und die Haube und der Deckel ausgebildet sind, bei in den Behälter eingesetzter Verpackung Haube und Klappdeckel miteinander zu verriegeln, sodass durch Öffnen des Klappdeckels zugleich die Haube der Verpackung öffenbar ist.

14. Vorrichtung nach Anspruch 13, bei dem die Schale und/oder die Haube ein Spritzgussteil ist.

15. Vorrichtung nach Anspruch 13 oder 14, bei dem die Haube eine Beschriftung und/oder Schale einen Farbcode trägt, wobei die Beschriftung die Größe der Reaktionsgefäße in der Verpackung angibt und/oder der Farbcode die Größe der Reaktionsgefäße in der Verpackung repräsentiert.

16. Vorrichtung nach einem der Ansprüche 8 bis 15, bei dem die Schale der Verpackung aus PE, PP oder COC besteht und/oder bei der die Abdeckung der Verpackung aus PE, PP oder COC besteht.

17. Vorrichtung nach einem der Ansprüche 1 bis 16, bei dem die Verpackung einen Feuchtigkeitsindikator zur Detektion der Restfeuchte aufweist und/oder bei dem die Verpackung einen Autoklavierindikator zur Detektion eines Autoklavierens unter vorgegebenen Bedingungen aufweist.

18. Verwendung des Systems nach einem der Ansprüche 1 bis 17, bei dem die Verpackung und die darin enthaltenen Reaktionsgefäße autoklaviert werden, wobei vorzugsweise die Verpackung mit den autoklavierten Reaktionsgefäßen in den Behälter eingesetzt wird, die Verpackung geöffnet wird und nach teilweiser Entnahme von Reaktionsgefäßen aus der Verpackung der Deckel des Behälters geschlossen wird.

19. Verwendung nach Anspruch 18, bei der die Verpackung gemäß einem der Ansprüche 1 bis 12 nach Entnahme sämtlicher Reaktionsgefäße nicht wiederverwendet wird oder bei der die Verpackung nach einem der Ansprüche 13 bis 15 nach Entnahme sämtlicher Reaktionsgefäße erneut mit Reaktionsgefäßen befüllt und wiederverwendet wird.
